# EUROPEAN PATENT APPLICATION

(11) **EP 1 111 061 A1**
(43) Date of publication of application: **27.06.2001**
(21) Application number: 99870268.2
(22) Date of filing: 20.12.1999
(51) Int. Cl.: C12N 15/65, C12N 15/70, C12N 15/90, C12N 1/21, C12N 5/10, A01K 67/027

(54) **Double selection vector**

(71) Applicant: UNIVERSITE LIBRE DE BRUXELLES, B-1050 Bruxelles (BE)
(72) Inventor: Gabant, Philippe, 1210 Brussels (BE); Szpire, Claude, 1410 Waterloo (BE)
(74) Representative: Van Malderen, Joelle

(57) **Abstract**

The present invention is related to a double selection vector (1) able to transform a cell of a specific organism which contains two different genes (2 and 3) comprising several cloning sites and encoding two different toxic molecules (4 and 5) to a procaryote cell, said genes being disposed in opposite lecture orientation to each other, upstream and downstream a cassette sequence (6). The present invention is also related to the procaryote host cell and to a method for the genetic modification of a cell or an organism comprising the use of said double selection vector.

## Description

### Field of the invention

The present invention is related to a nucleic acid construct to be incorporated in a double selection vector, its host cell and its use for the modification, the deletion or the replacement of genes in order to obtain non-human genetically modified unicellular or pluricellular organisms, preferably knock-in or knock-out organisms such as a knock-in or knock-out mouse.

### Background of the invention

Plasmid cloning vectors are commonly used for the propagation and amplification of DNA fragments in *Escherichia coli*. The insert of interest is ligated with the plasmidic vector linearized by a restriction endonuclease generating compatible ends. After transformation with ligation mix, experimentators face the problem of separating transformants that acquired a recombined plasmid from bacteria that contain the parental vector. To minimize the number of transformants due to self-ligated plasmid molecules, positive selection vectors were developed (for example, see Vernet et al., 1985 ; Kuhn et al., 1986 ; Gossen et al., 1992 ; Guilfoyle and Smith, 1994; Henrich and Schmidtberger, 1995 ; Trudel et al., 1996). Efficient positive selection cloning vectors were developed using *ccdB* (control of cell death B), a gene of the F plasmid coding for a natural poison acting on the *E. coli* gyrase (Bernard et al., 1994 and US-5,910,438). The *ccd* operon belongs to the poison-antidote systems found in different natural large plasmids (Jensen and Gerdes, 1995 ; Couturier et al., 1998). These poison-antidote loci (also termed post-segregational killing systems) contribute to the stability of low copy number plasmids in bacterial populations by killing daughter cells that have not inherited a plasmidic copy.

The main advantages of the *CcdB*-containing vectors over the other positive selection systems are i) the small size of their selective gene (C*cdB* : 303 bp), ii) the fact that the vector can be amplified in a host harboring a mutation that confers total resistance to the CcdB poison (gyrA462 resistant strain ; Bernard and Couturier, 1992). Since *E. coli* is the host used for most molecular cloning strategies, it is important to develop new systems which can enrich and widen the range of cloning possibilities. The positive selection technology using *CcdB* has been used to derive new vectors adapted to peculiar purposes: PCR cloning vectors (Gabant et al., 1997), vectors adapted for bacterial genetics (Gabant et al., 1998), and recently vectors designed for the construction and expression of epitope tagged proteins (Van Reeth et al., 1998).

In the field of genomics, a lot of new genes are cloned and sequenced. In order to assess the biological functions of these new coding sequences, their corresponding genes must be specifically mutated (deleted or modified) in the genome of an organism (for instance in a knock-out mouse), which allows the study of phenotype(s) related to the mutation introduced. The specificity of the mutation is given by a targeting vector (constructed in *E. coli*) containing homologous recombination arms.

However, the construction in *E*. *coli* of a vector containing recombination arms for homologous recombination before transfection into pluripotent cells or somatic cells can present several drawbacks. It is a long and complicated proceeding that requires several cloning steps for the insertion of said homologous recombination arms on each side of the cassette nucleotide sequence to be inserted in said pluripotent or said somatic cell.

### Aims of the invention

The present invention aims to provide a new nucleic acid construct to be inserted in a vector, also hereafter called "double selection vector" as well as its host cell, that does not present the drawbacks of the state of the art.

A particular aim of the present invention is to provide a nucleic acid construct that allows rapidly the cloning of a vector comprising said nucleic acid construct and having incorporated in correct orientation recombination arms, used in homologous recombinations allowing a modification, a replacement or a deletion of a target nucleotide sequence from the genome of a totipotent or a somatic cell.

Another aim of the present invention is to provide the corresponding cell wherein said target nucleotide sequence has been modified, replaced or deleted and possibly the corresponding organism made of or having incorporated said cell(s) and comprising in its genome the modification, the replacement or the deletion of said specific target nucleotide sequence.

### Summary of the invention

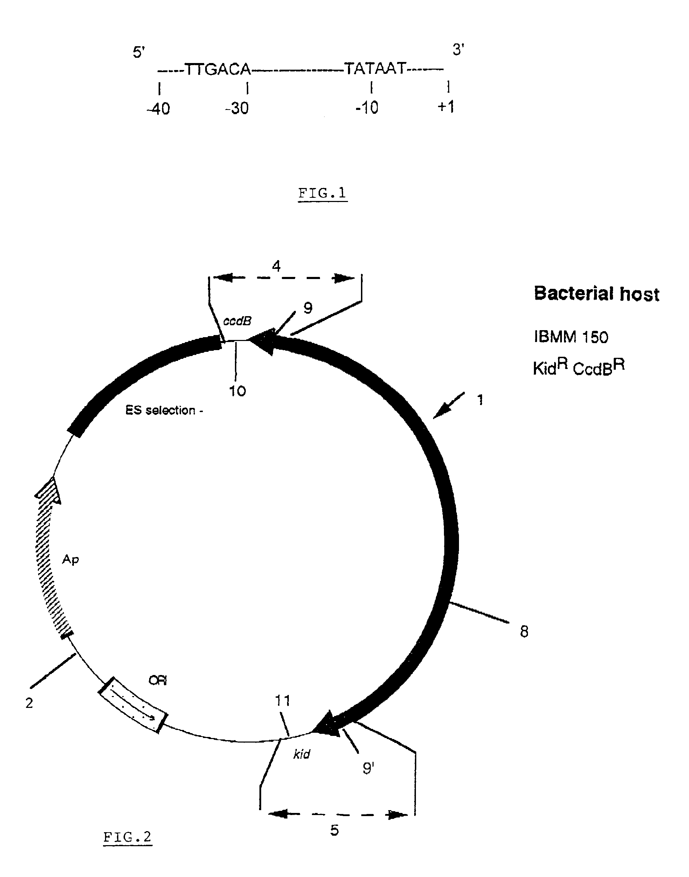

The present invention is related to a nucleic acid construct (1) to be incorporated in a double selection vector (2) able to transform a cell (3) of a specific organism, said construct (1) containing two different genes (4 and 5), each encoding a different toxic molecule (6 or 7) to a procaryote cell, preferably to *E*. *coli*, said genes (4 and 5) being disposed upstream and downstream a cassette sequence (8) (preferably being disposed in opposite and confluent lecture orientations to each other upstream and downstream said cassette sequence (8), as illustrated in the enclosed Fig. 1). Said nucleic acid construct comprises specific sequence portions allowing the inactivation of said genes, preferably through insertion in said genes or through a partial or total deletion of said genes.

According to a first embodiment of the present invention, the specific portions of the nucleic acid construct allowing the activation of genes are sequences which comprise several or unique cloning sites present in said genes, allowing the cleavage by restriction enzymes and allowing the insertion inside said genes of one or more foreigner sequences such as a recombination arm (15, 16) as described hereafter.

According to a second preferred embodiment of the present invention, the specific sequence portions could be sequences including specific sites recognised by recombinases (i.e. sites disposed upstream and downstream the genes or the portions of the genes to be deleted and recognised by said specific recombinase), and are for instance "ATT phage λ base sites" (specific recombination such as the one described by Ptashne.M (Genetic Switch, Cell Press, Cambridge 1992)). Preferably, said "ATT sites" are integrated in the nucleic acid construct according to the invention according to the method described by Landy A. (Annual Review Biochemistry, Vol. 58, p. 913 (1989)). Preferably, said ATT sites integrated in the nucleic acid construct comprise 25 base pairs and have the following genetic sequence SEQ ID N°1: ACA AGT TTG TAC AAA AAA GCA GGC T as well as its complementary strand or the sequence SEQ ID N°2: ACC CAG CTT TCT TGT ACA AAG TGG T and its complementary strand.

It is also possible to obtain a nucleic acid construct which may comprise more different specific sequence portions which can be inactivated by one or more of the above-identified methods (inactivation through an insertion in the genes or through a partial or total deletion of the genes).

"A gene (4 or 5) encoding a toxic molecule (6 or 7) for a procaryote cell" means a nucleotide sequence comprising a procaryote (preferably *E. Coli)* promoter/ operator sequence (9 or 9') and a sequence (10 or 11) encoding said toxic molecule (6 or 7) for said procaryote cell, preferably for *E*. *Coli.*

Each different gene comprised in said nucleic acid construct contains said specific sequence portions allowing the gene inactivation either in the procaryote promoter/operator sequence (9 or 9') or into the sequence (10 or 11) encoding the toxic molecule (6 or 7) for said procaryote cell. Said specific sequence portions present in each gene upstream or downstream said genes are different for each gene.

"Two different genes (4 and 5) encoding two different toxic molecules (6 and 7)" means that said two toxic molecules are able to reduce the growth or are able to kill said procaryote cell and are independent from each other (i.e. their modes of action are obtained by two different biological mechanisms), which results in the killing of said procaryote cell or affects its growth.

"A procaryote promoter/operator sequence (9 or 9')" is a regulatory sequence obtained from a procaryote cell, preferably from *E. Coli*, and comprising preferably the nucleotide sequence described in Fig. 1 for sigma 70 *E. coli* promoter.

According to the invention, the two toxic molecules (4 and 5) for a procaryote cell, are advantageously two poison proteins (encoded by a wild type or modified nucleotide sequence) which are naturally or artificially poisonous and affect one or more vital functions of a procaryote cell, preferably *E. Coli.*

A protein poison is also characterised by the existence of an antidote or an anti-poison such as the protein CcdB and its antagonist CcdA, the protein Kid and its antagonist Kis, the protein Doc and its antagonist Phd, the protein HoK and its antagonist SoK, relE toxin and its anti-toxin RelB, PasA and its antidote PasB and PasC, mazE and its antidote mazF, and other poison molecules which are or are not of plasmid origin.

Other examples of toxic molecules for a procaryote cell are the protein encoded by the gene sacB (from Bacillus amylolique-faciens), the protein GpE, the protein GATA-1 or the protein Crp. The gene sacB encodes the levan sucrase which catalyzes the hydrolysis of sucrose into products which are toxic for *E*. *Coli* (Pierce et al. *Proc. Natl. Acad. Sci*., Vol. 89, N°6 (1992) p. 2056-2060). The protein GpE encodes the E genes from the bacteriophage ϕX174 which includes six unique restriction sites and encodes gpE and which causes lysis of *E. Coli* cell (Heinrich et al., Gene, Vol. 42 n°3 (1986) p. 345-349). The protein GATA-1 has been described by Trudel et al. (*Biotechniques 1996*, Vol. 20(4), p. 684-693). the protein Crp has been described by Schlieper et al. *(Anal. Biochem. 1998,* Vol. 257(2), p. 203-209).

"A cassette sequence (8)" is a sequence of DNA containing one or more selectable genes expressed in the eucaryote cell. the insertion of such sequence into the genome of the cell confers to this organism a selectable property (for example the resistance to an antibiotic like Neo giving a resistance to Geneticin). Said cassette may further contain sequences to be expressed in the host cell, allowing the detection of cells by reporter genes (such as LacZ, GFP, etc.) or specific recombinases (such as Cre of P1). This cassette may also contain site allowing total or partial deletion(s) of DNA by action of recombinases (such as LoxP sites for Cre). Finally, this cassette may also consist or contain any coding sequence expressed in the eucaryote after recombination.

Preferably, the nucleotide sequence (10 or 11) incorporated in the two genes (4 and 5) correspond to the following sequences SEQ ID NO. 1 and SEQ ID NO. 2, comprising respectively two fusion proteins poisons made of a polylinker (12) comprising several unique cloning sites different for each sequence and a nucleotide sequence (13, 14) encoding either the protein poison CcdB or the protein poison Kid.

Another aspect of the present invention is the vector (2) (preferably a viral or plasmid vector such as the pUC18 vector) comprising said nucleic acid construct and all the necessary elements for the replication of said vector in a procaryote cell, preferably in *E. Coli.*

A further aspect of the present invention is related to the procaryote cell transformed by said vector, preferably a procaryote cell "which is resistant to one of the two toxic molecules (6 and 7) expressed by said vector". Preferably, said procaryote cell (IBMM140) comprises a gene encoding an antidote to one of said toxic (poison) molecules, preferably a gene encoding the molecule Kis which is the antidote to the protein Kid or to a fusion protein comprising said protein Kid.

The present invention is also related to the host cell of said nucleic acid construct and said double selection vector, preferably a procaryote cell, which is resistant to the two toxic molecules (6 and 7) encoded by the two genes (4 and 5). Preferably, said procaryote cell possesses mutation(s) that confers a resistance to the toxic activity of said first and/or said second toxic molecule(s) and or possesses one or more genes that encode one or more antidote(s) to said first and/or said second toxic molecule(s).

Advantageously, said procaryote host cell (IBMM150) having the deposit number LMGP-19171 (filed on November 29, 1999) possesses a mutation wherein the Arginine 462 is replaced by a Cysteine in the amino acid sequence of the GyrA polypeptide of the gyrase (see US patent 5,910,438) and possesses the genetic sequence encoding the protein Kis that is an anti-poison of the protein Kid.

According to another embodiment of the invention, the double selection vector according to the invention further comprises, inserted in one of the cloning sites of the first gene (4), a first recombination arm (15) and at one of the cloning sites of the second gene (5), a second recombination arm (16).

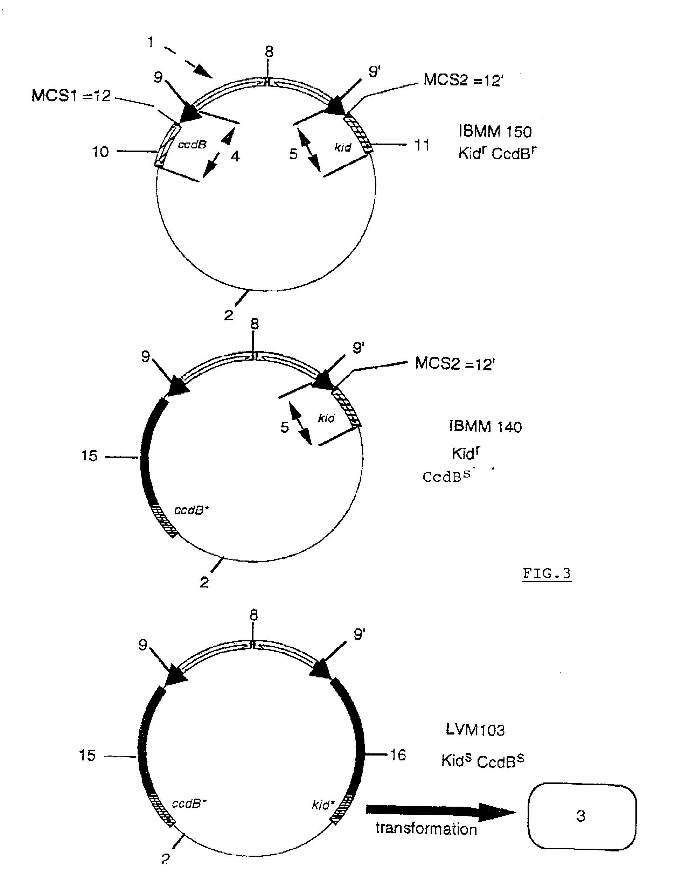

Said double selection vector having incorporated said recombination arms, hereafter called "targetor vector", allows the insertion of a portion (comprising the cassette sequence (8)) of the nucleic acid construct (1) according to the invention in the genome of a transformed cell (3). The "targetor vector" having integrated the recombination arms (15 and 16) in a correct orientation in the nucleic acid construct (1) according to the invention (after its cloning in *E. coli*) is selected and recovered. Thereafter, said "targetor vector" is used to obtain an homologous recombination between the two recombination arms (15 and 16) and corresponding nucleotide sequences present in the genome of the cell, said sequences being disposed upstream and downstream the target sequence to be modified, deleted or replaced (as illustrated in Fig. 2).

Therefore, a further aspect of the present invention is related to the cell (3) (totipotent cell or somatic cell), preferably a pluripotent embryonic stem (ES) mice cell, in which a target nucleotide sequence has been modified, deleted or replaced by the portion (comprising the cassette sequence (8)) of the "targetor vector" according to the invention

Another aspect of the present invention is related to the corresponding non-human pluricellular organism (selected from the group consisting of micro-organisms, fungi (including yeasts), plants or non-human animals (preferably a mammal such as a mouse) made of said cells or having incorporated said cells (for instance in a chimera) and comprising in its genome the above-described deletion or modification of a target nucleotide sequence. Said cell and non-human organism can be identified by a genetic analysis of the incorporated foreigner genetic fragments (i.e. fragments of the nucleic acid construct (1) according to the invention), especially one or more procaryote promoter/operator sequences (9 or 9'), preferably obtained from *E*. *coli*, in particular procaryote promoter/operator sequences (9) disposed in opposite divergent lecture orientation upstream and downstream a cassette sequence (8).

A further aspect of the present invention is related to a method for the replacement of a target genetic sequence into a cell (3) by a cassette sequence (8), comprising the steps of:
- incorporating the nucleic acid construct (1) according to the invention into a vector (2),
- possibly submitting said vector (2) (if it is circular) to an enzymatic action upon the specific sequence portions (preferably to the action of a restriction enzyme upon the unique cloning site (12) of the nucleic acid construct (1) incorporated in said vector), allowing the cleavage or the partial or total deletion of the first gene (4),
- incorporating into said vector (2) a first recombination arms (15) that desactivates the toxic activity of the nucleotide sequence (10) present in the first gene (4),
- selecting the vector having integrated in the correct orientation the first recombination arm (15) (by transforming a strain (IBMM140) which is sensible to the toxic activity of the first gene (4) but which is resistant to the toxic activity of the second gene (5)),
- submitting the recovered vector to an enzymatic action upon the specific sequence portions (preferably to the action of a restriction enzyme upon the unique cloning site (12') of the nucleic acid construct (1) incorporated in said vector), allowing the cleavage or the partial or total deletion of the second gene (5),
- incorporating into said vector (2) a second recombination arm (16) that desactivates the toxic activity of the nucleotide sequence (11) present in the second gene (5),
- selecting the vector having integrated in the correct orientation the second recombination arm (16) (by transforming a strain that is sensible to the toxic activity of the second gene (5)),
- cloning said vector (2) in a procaryote cell, preferably in *E*. *coli*, and
- transforming a totipotent or a somatic cell (3) in conditions allowing the replacement of a target nucleotide sequence present in the genome of said cell by the cassette sequence (8) of the nucleic acid construct (1) present in said vector (2) by homologous recombination between the arms (15 and 16) and corresponding nucleotide sequences present upstream and downstream the target sequence to be modified or deleted in the genome of said cell (3),
- recovering the somatic or totipotent cell (3) wherein the target sequence has been replaced by the cassette sequence (8), and
- possibly obtaining a pluricellular organism made of said cell (3) or having incorporated said cell (3) and comprising in its genome the deletion or the modification of the target nucleotide sequence, replaced by the cassette sequence (8) of the nucleic acid construct (1) according to the invention.

The present invention is also related to a kit of parts comprising one or more of the above-described products, preferably the nucleic acid construct (1) according to the invention, advantageously incorporated in the vector (2) according to the invention. Said vector is preferably linear and has been cleaved by a restriction enzyme upon one of the unique cloning sites (12) of the nucleic acid construct (1) incorporated in said vector (2). The kit according to the invention comprises also a procaryote cell for said vector, preferably a procaryote host cell, that is resistance to the toxic activity of the second toxic molecule (7) or possesses a gene that encodes one or more antidotes to said second toxic molecule (7), said second toxic molecule (7) being encoded by the gene (5) that has not been cleaved by the restriction enzyme above-described.

Preferably, said procaryote cell is the strain IBMM140 that is resistant to the Kid protein and is competent (able to receive DNA under the form of the vector according to the invention). This competence can be obtained by a chemical treatment or by a proceeding allowing electroporation of the strain( such as the one described by Ausubel et al. (Current Protocols in Molecular Biology, New York, Greene Publishing Associates and Whiley-Interscience (1994)).

Furthermore, said sensibility to the toxic activity of the second toxic molecule (7) encoded by the gene (5) is advantageously inducible by a metabolite. For instance the strain IBMM140 allows the production of the antidote Kis in the presence of an increased concentration of arabinose.

The kit according to the invention may further comprise culture medium (under solid or liquid form) and other means for improving the specific method according to the invention (for instance sequencing and/or amplification primers, restriction or recombinase (clonase™ mix from Gibco-BRL) enzymes, DNA polymerase, DNA ligase, etc.) as well as other specific saline solution buffers containing the nucleotides, control plasmids and specific eucaryotic cells to be transformed (for instance ES cells).

### Short description of the drawings

Figure 1 represents a promoter consensus sequence defined for a sigma 70 *E. coli* promoter.

Figure 2 represents the nucleic acid construct according to the invention integrated into a plasmid.

Figure 3 represents schematically several steps used in the method according to the invention for the replacement of a target genetic sequence into a cell by a cassette sequence.

### Detailed description of the invention

### Description of plasmids and strains

The nucleic acid construct 1 comprises, preferably integrated into a plasmid 2 (for instance of the type pUC18), two positive selection sequences (corresponding to the above-described genes 4 and 5) disposed preferably in opposite divergent lecture orientation upstream and downstream a cassette sequence 8. The first positive selection sequence 4 incorporating the CcdB sequence has been described by Bernard P. et al. (Gene 148, pp. 71-74 (1994)). The other positive selection sequence 5 is based upon the use of the *parD* killer gene encoding the *kid* poison toxin.

The *kis* coding sequence was amplified by PCR on the plasmid R1 drdl9 (Meynell and Datta, 1966), using the following primers: kis1-5'gaggaattctggaggtgaagaatgcatac3'- and kis2-5 'gagaagctttcagatttcctcctgaccag3'-. The resulting product was cloned in TOPO activated pCR-XL TM vector (Invitrogen, Carlsbad, CA, USA), and this insert was then subcloned in pBAD33 (Guzman et al., 1995) to give pBAD-*kis*. To construct λ : : *kis*, the *kis* gene, placed under the control of *Pbad* promoter was amplified by PCR from pBAD-*kis* using the following primers: λ1 -5'tagagatctgatgcataatgtgcctgtc3'- and λ2 : -5'tagagatctgagcaaaaacaggaaggc3'-. The resulting product was cloned in TOPO activated pCR-XL. This insert was then cloned in pRS551 (Simons, et al., 1987) open by *BaimH*I to generate pRS*-kis*. The MM294 (Backman and Boyer, 1983) strain containing pRS*-kis* was infected by λRS45 and recombinants were selected on LB-kanamicyn as described by Simons, et al., (1987) in LVM103 F' traD36 *proAB lacI*^{*q*} *IacZΔ* M15/Δ*ara leu* : :Tn*10 supE thi-1* Δ(*lac-proAB*). This strain, lysogenized by λ::*kis* was called IBMM120. To construct Tn::*kis*, the *kis* gene placed under the control of the *Pbad* promoter and the chloramphenicol resistance gene were amplified by PCR from pBAD-*kis* using the following primers: Tnl -5'taggcggccgcgatgcataatgtgcctgtc3'- and Tn2: -5'taggcggccgccagaagccactggagcacc3'-. The resulting product was cloned in TOPO activated pCR-XL and subcloned in the unique *Sfi*I site of the suicide vector pMF100, a Δ Km derivative of pUT/Km (Herrero et al., 1990). The pUT-*kis* plasmid was transformed in S17-1 (λ-pir) : *pro*82 *rfb*D1 *spoT1 supE44 endAl hsdR17 recA* [RP4-2-Tc : :Mu-Km : :Tn7] (λ-pir) (de Lorenzo via Van der Lelie). This strain was mated with IBMM139 (LVM103 λ resistant) and a clone, IBMM140, with a Tn::*kis* transposed in the chromosome, was isolated.

### Construction of the vectors pKID18 and pKID19

*parD* is a poison-antidote stabilization system of the R1 plasmid, a member of the IncFII plasmid family. This locus participates in the maintenance of R1 by postsegregational killing of plasmid-free bacteria and consists in a small operon containing two genes: *kid* (333bp) and *kis* (258bp) coding for a killer component (Kid) and its antagonist (Kis), respectively (Bravo et al., 1988). This system is perfectly conserved and functional in another IncFII plasmid, R100 (pem system: Tsuchimoto et al., 1988), which contains the genes termed *pemI* (identical to *kis*) and *pemK* (identical to *kid*). The structure and function of parD are similar to those described for *ccd* of the F plasmid. Using the R1 *kid* killer gene, we constructed new cloning vectors allowing positive selection of recombinants. The *kid* sequence was fused in frame with the multiple cloning site (MCS) of either pUC18 or pUC19 (Yanish-Perron et al., 1985) to generate pKID18 and pKID19 respectively.(comprising the enclosed sequence SEQ ID NO1)

Both pKID18 and pKID19 express a fused Kid protein under the control of the lactose promoter (*Plac*). These plasmids were amplified in TOP10F' bacteria *[F' lacI*^{*q*} Tc ^{R}/*mcrA* Δ(*mrr-hsd*RMS*-mcr*BC) φ80 *lacZΔ* M15 *Δlac*X74 *deoR recA*1 *ara*D139 *Δ*(*ara-leu*) 7697 *galU galK rpsL endA1 nupG*] (Invitrogen, Carlsbad, CA, USA). In this strain, the *kid* fusions are transcriptionally repressed by the host *lacI*^{*q*} repressor.

In order to avoid any selective advantage for inactive mutants in the *kid* selective marker during amplification, it is essential to amplify these vectors in a host insensitive to Kid poisoning. Bacterial strains expressing Kis are host for *kid*-based vector amplification.

Two strategies may be used: the *kis* gene was inserted into a lambda vector (λ: :*kis*) or into a one hooper transposon (Tn: :*kis*) carried by a suicide vector. Strains lysogenic for λ: :*kis* or containing a copy of Tn: :*kis* in the chromosome were isolated. The two resulting strains IBMM120 and IBMM140 were shown to be completely resistant to pKID18/19 poison activity upon induction of the chromosomal *kis* gene.

### pKID vectors and positive selection

The insertion of a DNA fragment into the multiple cloning site of pKID18/19 leads to disruption of *kid* genetic information and thus to inactivation of the toxin. Consequently, only bacteria harboring recombinant plasmids should give rise to viable colonies, whereas those bearing the parental vectors should be killed.

### CcdB and Kid selections are independent

In order to determine whether CcdB and Kid poisoning are independent, one may determine whether the B462 strain coding for a CcdB insensitive gyrase and therefore resistant to CcdB killing, is killed by fused Kid proteins and vice-versa, whether the IBMM120 and IBMM140 strains resistant to Kid upon induction of *kis*, are killed by fused CcdB proteins. Results show that the CcdB insensitive gyrase (gyrA462) does not protect against Kid poisoning and synthesis of Kis (IBMM120 and IBMM140) does not prevent the killing by CcdB. A double resistant strain was constructed by introducing the gyr462 mutation (CcdB^{R}) (see US-5,910,438) into the IBMM140 strain (Kid^{R}). This new strain called IBMM150 and having the deposit number LMGP-19171 was shown to resist to both systems and thus to constitute the ideal host for the amplification of vectors based either on *ccdB*, on *kid* or on both systems.

The two above-identified positive selection sequences or genes 4 and 5 according to the invention are incorporated in a plasmid for obtaining the vector 2 as represented in Fig. 1. Said nucleic acid construct is preferably incorporated into a plasmid of the type pUC18 (Yanish-Perron et al., Gene 33, pp. 103-109 (1985)) cleaved by the enzyme Aat2 and AflIII wherein the specific nucleotide sequences 10 and 11 encoding the poison protein CcdB and Kid are introduced after their amplification by PCR. Between said poisonous genes 4 and 5 is cloned a cassette sequence 8 according to the following method. Advantageously, the two multiple cloning sites (MCS) 12 and 12' in said positive selection sequences (or genes 4 and 5) comprise very rare enzyme restriction sites and comprise also blunt sites that allow insertion of DNA fragments generated by genetic amplification, preferably by PCR. Examples of said enzymes are SwaI, PmeI and SrfI.

In addition, the vector 2 according to the invention comprises also the specific enzyme restriction sites NotI and SfiI integrated between one of the nucleotide sequences 10 and 11 encoding a poison protein and the cassette sequence 8 for a negative selection.

### Use of the targeting vector according to the invention in the preparation of a knockout mouse

A clone containing a 129 genomic fragment of AFP loci was isolated from a lambda library. The library was screened with a probe containing the mouse Afp promoter. The genomic insert of about 16 kb was subcloned in the vector 2 according to the invention having incorporated as a cassette sequence the IRES lacZ/neo reporter-selective cassette 8. The obtained vector is also incorporating two recombination arms 15 and 16. The 5' arms (2.5 kb) was generated by polymerase chain reaction (PCR) using the following primers: N-Merl: agagcggccgcggaagtgacaaagcagaacc annealing to the MerI sequence of the Afp enhancer 1 (Godboute et al. (1988)) and a primer of the X-exonl: agactcgagggatgagggaagcgggtgtg complementary to the afp exonl. The PCR fragment generated using Pfu polymerase (Stratagene) was cloned in the pCR-blunt vector (Invitrogen).

The 3' arms was subcloned from the lambda into pBSIIKS+ vector (Stratagene). The 5' recombination arms was introduced upstream the 3' recombination arms. This construction was electroporated into E14 ES cells 3. Correctly targeted clones were identified by Southern blot analysis using an external probe from the 5' region.

### ES cell injections and animal genotyping

Recombinant ES cells 3 carrying the targeted allele were injected in C57BL/6J blastocysts. Animals were genotyped by extraction of DNA from tails.

### RNA isolation, Northern blot analysis

Total RNA was isolated using Trizol (Gibco BRL) extraction according to the manufacturer instructions. For the Northern analysis 20µg of total RNA were electrophoresed and transferred to nylon membranes as described. Filters were then hybridized.

### Western blot analysis

Proteins were separated by SDS-PAGE using 7.5% polyacrylamide gels in a Bio-Rad Mini Protean gel chamber and blotted onto Nitrocellulose filters in a Bio-Rad Trans Blot chamber according to the manufacturer's instructions. Proteins were detected using anti-AFP, anti-Albumin; anti Betagalactosidase serum (ICN Biochemicals) the signal was detected with ECL detection system (Amersham).

### LacZ reporter gene expression

To isolate embryonic stages, natural matings were set up and presence of a vaginal plug at noon the following day was taken as 0.5 days of gestation. Staged embryos were stained with X-Gal as wholemounts as described by Forrester et al. (1996). For cryostat sectioning, tissues were embedded in optimal cutting temperature (OTC) compounds (Miles, Inc., Elkart, IN), and sections stained for X-Gal were counterstained with haematoxylin and eosin, and mounted.

### Targeted mutagenesis of the Afp gene

The *Afp* gene was disrupted by gene targeting in embryonic stem (ES) cells. The *lacZ* reporter was introduced in *Afp* gene by homologous recombination and placed under the control of the AFP promoter-enhancer region. The resulting allele is deleted for most of the sequence of exon1, for exon2 and 3 and homologous insertion was detected by Southern analysis. To test the functionality of the reporter one may take advantage of the observation that AFP is expressed in embryoid bodies (Abe et al., 1996). Reporter gene activity is highly turn on in some cells of these bodies.

ES cells *Afp* lacZ1/+ were injected into C57BL/6J blastocysts. Chimeric animals were obtained and mated with outbred CD1 or inbred 129/CGR to test for germ line transmission. Phenotypically normal heterozygous mice afp lacZl/+ were generated and detected by Southern blot.

The strain IBMM150 has been submitted to a deposit according to the Budapest Treaty under the deposit number LMGP-19171 (filed on November 29, 1999) at the Belgian Coordinate of Micro-organisms BCCM-LMGP, Laboratorium voor Microbiologie-Bacterienverzameling, Universiteit Gent, K.L. Ledeganckstraat 35, B-9000 GENT-BELGIUM.
the vectors pKID18/19 comprisse the following sequence
SEQ ID N01 which is a sequence coding for a fusion poison
protein made of a polylinker and the coding sequence kid :

SEQ ID N01 :

1 atg acc atg att acg aat tat tta aat cgc gag tcg acg gcc
Met Thr Met Ile Thr Asn Tyr Leu Asn Arg Glu Ser Thr Ala

43 gca gtg gcc agc tta ttg gaa aga ggg gaa atc tgg ctt gtc
Ala Val Ala Ser Leu Leu Glu Arg Gly Glu Ile Trp Leu Val

85 tcg ctt gat cct acc gca ggt cat gag cag cag gga acg cgg
Ser Leu Asp Pro Thr Ala Gly His Glu Gln Gln Gly Thr Arg

127 ccg gtg ctg att gtc aca ccg gcg gcc ttt aat cgc gtg acc
Pro Val Leu Ile Val Thr Pro Ala Ala Phe Asn Arg Val Thr

169 cgc ctg cct gtt gtt gtg ccc gta acc agc gga ggc aat ttt
Arg Leu Pro Val Val Val Pro Val Thr Ser Gly Gly Asn Phe

211 gcc cgc act gcc ggc ttt gcg gtg tcg ttg gat ggt gtt ggc
Ala Arg Thr Ala Gly Phe Ala Val Ser Leu Asp Gly Val Gly

253 ata cgt acc aca ggt gtt gta cgt tgc gat caa ccc cgg aca
Ile Arg Thr Thr Gly Val Val Arg Cys Asp Gln Pro Arg Thr

295 att gat atg aaa gca cgg ggc gga aaa cga ctc gaa cgg gtt
Ile Asp Met Lys Ala Arg Gly Gly Lys Arg Leu Glu Arg Val

337 ccg gag act atc atg aac gaa gtt ctt ggc cgc ctg tcc act
Pro Glu Thr Ile Met Asn Glu Val Leu Gly Arg Leu Ser Thr

379 att ctg act tga
Ile Leu Thr ---

### REFERENCES

- Backman, K. & Boyer, H.W., *Gene* **26**, pp. 197-203 (1983)
- Bernard P., *BioTechniques* **21,** pp. 320-323 (1996)
- Bernard P. et al., *Gene* **148,** pp. 71-74 (1994)
- Bravo, A. et al., *Mol. Gen. Genet.* **215,** pp. 146-151 (1988)
- Couturier, M. et al., *Trends in Microbiology **6,*** pp. 269-275 (1998)
- Gabant, P. et al., *BioTechniques* **23,** pp. 938-941 (1997)
- Gabant, P. et al., *Gene* **207,** pp. 87-92 (1998)
- Gossen J.A. et al., *Nucleic Acids Res.* **20,** p. 3254 (1992)
- Guilfoyle R.A. & Smith L.M., *Nucleic Acids Res.* **22,** pp. 100-107 (1994)
- Guzman L.M. et al., J. *Bact.* **177**, pp. 4121-4130 (1995)
- Herrero M. et al., J. *Bact.* **172,** pp. 6557-6567 (1990)
- Jensen, R.B. & Gerdes, K., *Mol. Microbiol.* **17**, pp. 205-210 (1995)
- Kuhn I. et al., *Gene* **42**, pp. 253-263 (1986)
- Meynell, E. & Datta, N., *Gen. Res.* **7,** p. 141 (1966)
- Ruiz-Echevarria M.J. et al., *J. Mol. Biol.* **7,** pp. 568-577 (1995)
- Simons R.W. et al., *Gene* **53,** pp. 85-96 (1987)
- Trudel P. et al., *BioTechniques* **20,** pp. 684-693 (1996)
- Tsuchimoto, S. et al., *J. Bact.* **170,** pp. 1461-1466 (1988)
- Van Reeth, T. et al., *BioTechniques **25,*** pp. 898-904 (1998)
- Vernet T. et al., *Gene **34,*** pp. 87-93 (1985)
- Yanish-Perron, C. et al., *Gene **33,*** pp. 103-109 (1985)

## Claims

1. Nucleic acid construct (1) to be incorporated in a double selection vector (2) able to transform a cell (3) of a specific organism, characterised in that said construct (1) contains two different genes (4 and 5), each gene encoding a different toxic molecule (6 and 7) to a procaryote cell, preferably to *E*. *Coli*, said genes (4 and 5) being disposed upstream and downstream a cassette sequence (8) and in that said nucleic acid construct comprises specific sequence portions allowing inactivation of said genes.

2. Nucleic acid construct according to claim 1, characterised in that the genes inactivation is obtained through an insertion of a foreigner sequence in said genes or through a partial or total deletion of said gene(s).

3. Nucleic acid sequence according to claim 1 or 2, wherein each gene (4 or 5) comprises a procaryote promoter/operator sequence (9 or 9') and a sequence (10 or 11) encoding a toxic molecule (6 or 7) for said procaryote cell.

4. Nucleic acid construct according to claim 1 to 3, wherein said genes are disposed in opposite and confluent lecture orientation to each other upstream and downstream the cassette sequence (8).

5. Nucleic acid construct according to any one of the preceding claims, wherein each sequence (10 or 11) encoding a toxic molecule (6 or 7) to a procaryote cell is a nucleotide sequence which encodes a fusion protein active as a poison to the procaryote cell and made of a coding nucleotide sequence which comprises several unique cloning sites (12) and a nucleotide sequence encoding a protein poison.

6. Nucleic acid construct according to claim 5, wherein the protein poisons are respectively the protein CcdB and the protein Kid.

7. A vector able to transform a cell having incorporated the nucleic acid construct according to any one of the preceding claims.

8. A vector according to claim 7, further comprising, inserted in one of the unique cloning sites of the gene (4), a first recombination arm (15) and inserted in one of the unique cloning sites of the second gene (5) a second recombination arm (16), said recombination arms being able to allow an homologous recombination with corresponding sequences present upstream and downstream a target sequence to be deleted or modified and present in the genome of a cell.

9. A procaryote cell transformed by the vector according to claim 7.

10. A prokaryotic host cell for the vector according to claim 7, which possesses a mutation which confers resistance to the toxic activity of one or more of the toxic molecules (6 and 7) encoded by the two different genes (4 and 5) present in said vector and/or possesses one or more genes that encode one or more molecules which is/are anti-poison of one or more of said toxic molecules (6 and 7).

11. Cell according to claim 10, having the deposit number LMGP-19171.

12. Method for the modification or the replacement of a target genetic sequence into a cell (3) by a cassette sequence (8), comprising the following steps:
- incorporating the nucleic acid construct (1) according to anyone of the claims 1 to 5 into a vector (2),
- possibly submitting said vector (2) to an enzymatic action upon the specific sequence portions of said nucleic acid construct (preferably to the action of a restriction enzyme upon the unique cloning site (12) of the nucleic acid construct (1) incorporated in said vector), allowing the cleavage or the partial or total deletion of the first gene (4),
- incorporating into said vector (2) a first recombination arm (15) that desactivates the toxic activity of the nucleotide sequence (10) present in the first gene (4),
- selecting the vector having integrated in the correct orientation a first recombination arm (15) (by transforming a strain (IBMM140) which is sensible to the toxic activity of the first gene (4) but which is resistant to the toxic activity of the second gene (5)),
- submitting the recovered vector to an enzymatic action upon the specific sequence portions of said nucleic acid construct (preferably to the action of a restriction enzyme upon the unique cloning site (12') of the nucleic acid construct (1) incorporated in said vector), allowing the cleavage or the partial or total deletion of the second gene (5),
- incorporating into said vector (2) a second recombination arm (16) that desactivates the toxic activity of the nucleotide sequence (11) present in the second gene (5),
- selecting the vector having integrated in the correct orientation the second recombination arm (16) (by transforming a strain that is sensible to the toxic activity of the second gene (5)),
- cloning said vector (2) in a procaryote cell, preferably in *E*. *coli*, and
- transforming a totipotent or a somatic cell (3) in conditions allowing the replacement of a target nucleotide sequence present in the genome of said cell by the cassette sequence (8) of the nucleic acid construct (1) present in said vector (2) by homologous recombination between the arms (15 and 16) and corresponding nucleotide sequences present upstream and downstream the target sequence to be modified or deleted in the genome of said cell (3),
- recovering the somatic or totipotent cell (3) wherein the target sequence has been replaced by the cassette sequence (8), and
- possibly obtaining a pluricellular organism made of said cell (3) or having incorporated said cell (3) and comprising in its genome the deletion or the modification of the target nucleotide sequence, replaced by the cassette sequence (8) of the nucleic acid construct (1).

13. Cell or organism obtained by the method according to the claim 12 and having preferably incorporated into their genome fragments of the vector according to any of the preceding claims 1 to 6, said fragments being preferably two procaryote promoter/operator sequences, preferably from *E*. *Coli*, disposed in divergent and opposite lecture orientation.

14. Cell according to the claim 12, being a totipotent cell, preferably a pluripotent embryonic stem (ES) mice cell.

15. Non-human pluricellular organism comprising or made of the cell according to claim 13, preferably a non-human mammal, preferably a mouse.

16. Kit of parts comprising a linear or circular vector according to claim 7 or the vector according to claim 8, and means or media for performing the method for the modification or the replacement of a target genetic sequence into a cell by a cassette sequence according to the method of claim 11.


### Annex to the application documents - subsequently filed sequences listing

SEQUENCE LISTING

<110> UNIVERSITE LIBRE DE BRUXELLES

<120> DOUBLE SELECTION VECTOR

<130> P.ULB.51/EP

<140> 99870268.2
<141> 1999-12-20

<160> 10

<170> PatentIn Ver. 2.1

<210> 1
<211> 25
<212> DNA
<213> Artificial Sequence

<220>
<223> Description of Artificial Sequence: construct

<400> 1
acaagtttgt acaaaaaagc aggct 25

<210> 2
<211> 25
<212> DNA
<213> Artificial Sequence

<220>
<223> Description of Artificial Sequence: construct

<400> 2
acccagcttt cttgtacaaa gtggt 25

<210> 3
<211> 29
<212> DNA
<213> Artificial Sequence

<220>
<223> Description of Artificial Sequence: primer

<400> 3

gaggaattct ggaggtgaag aatgcatac 29

<210> 4
<211> 29
<212> DNA
<213> Artificial Sequence

<220>
<223> Description of Artificial Sequence: primer

<400> 4
gagaagcttt cagatttcct cctgaccag 29

<210> 5
<211> 28
<212> DNA
<213> Artificial Sequence

<220>
<223> Description of Artificial Sequence: primer

<400> 5
tagagatctg atgcataatg tgcctgtc 28

<210> 6
<211> 27
<212> DNA
<213> Artificial Sequence

<220>
<223> Description of Artificial Sequence: primer

<400> 6
tagagatctg agcaaaaaca ggaaggc 27

<210> 7
<211> 30
<212> DNA
<213> Artificial Sequence

<220>
<223> Description of Artificial Sequence: primer

<400> 7

taggcggccg cgatgcataa tgtgcctgtc                                    30

<210> 8
<211> 30
<212> DNA
<213> Artificial Sequence

<220>
<223> Description of Artificial Sequence: primer

<400> 8
taggcggccg ccagaagcca ctggagcacc                                    30

<210> 9
<211> 390
<212> DNA
<213> Artificial Sequence

<220>
<221> CDS
<222> (1)..(387)

<220>
<223> Description of Artificial Sequence: fusion posion
      protein nucleic acid sequence

<400> 9
atg acc atg att acg aat tat tta aat cgc gag tcg acg gcc gca gtg      48
Met Thr Met Ile Thr Asn Tyr Leu Asn Arg Glu Ser Thr Ala Ala Val
  1               5                  10                  15

gcc agc tta ttg gaa aga ggg gaa atc tgg ctt gtc tcg ctt gat cct      96
Ala Ser Leu Leu Glu Arg Gly Glu Ile Trp Leu Val Ser Leu Asp Pro
             20                  25                  30

acc gca ggt cat gag cag cag gga acg cgg ccg gtg ctg att gtc aca     144
Thr Ala Gly His Glu Gln Gln Gly Thr Arg Pro Val Leu Ile Val Thr
         35                  40                  45

ccg gcg gcc ttt aat cgc gtg acc cgc ctg cct gtt gtt gtg ccc gta     192
Pro Ala Ala Phe Asn Arg Val Thr Arg Leu Pro Val Val Val Pro Val
     50                  55                  60

acc agc gga ggc aat ttt gcc cgc act gcc ggc ttt gcg gtg tcg ttg     240
Thr Ser Gly Gly Asn Phe Ala Arg Thr Ala Gly Phe Ala Val Ser Leu
 65                  70                  75                  80

gat ggt gtt ggc ata cgt acc aca ggt gtt gta cgt tgc gat caa ccc    288
Asp Gly Val Gly Ile Arg Thr Thr Gly Val Val Arg Cys Asp Gln Pro
                85                  90                  95

cgg aca att gat atg aaa gca cgg ggc gga aaa cga ctc gaa cgg gtt    336
Arg Thr Ile Asp Met Lys Ala Arg Gly Gly Lys Arg Leu Glu Arg Val
            100                 105                 110

ccg gag act atc atg aac gaa gtt ctt ggc cgc ctg tcc act att ctg    384
Pro Glu Thr Ile Met Asn Glu Val Leu Gly Arg Leu Ser Thr Ile Leu
        115                 120                 125

act tga                                                            390
Thr

<210> 10
<211> 129
<212> PRT
<213> Artificial Sequence
<223> Description of Artificial Sequence: fusion posion
      protein nucleic acid sequence

<400> 10
Met Thr Met Ile Thr Asn Tyr Leu Asn Arg Glu Ser Thr Ala Ala Val
  1               5                  10                  15

Ala Ser Leu Leu Glu Arg Gly Glu Ile Trp Leu Val Ser Leu Asp Pro
             20                  25                  30

Thr Ala Gly His Glu Gln Gln Gly Thr Arg Pro Val Leu Ile Val Thr
         35                  40                  45

Pro Ala Ala Phe Asn Arg Val Thr Arg Leu Pro Val Val Val Pro Val
     50                  55                  60

Thr Ser Gly Gly Asn Phe Ala Arg Thr Ala Gly Phe Ala Val Ser Leu
 65                  70                  75                  80

Asp Gly Val Gly Ile Arg Thr Thr Gly Val Val Arg Cys Asp Gln Pro
                 85                  90                  95

Arg Thr Ile Asp Met Lys Ala Arg Gly Gly Lys Arg Leu Glu Arg Val
            100                 105                 110

Pro Glu Thr Ile Met Asn Glu Val Leu Gly Arg Leu Ser Thr Ile Leu
        115                 120                 125

Thr